(19) Europäisches Patentamt
European Patent Office
Office européen des brevets

(11) EP 4 406 647 B1

(12) **EUROPEAN PATENT SPECIFICATION**

(45) Date of publication and mention of the grant of the patent:
**12.11.2025 Bulletin 2025/46**

(21) Application number: **22872838.2**

(22) Date of filing: **16.09.2022**

(51) International Patent Classification (IPC):
***B01J 31/24*** (2006.01) ***C07B 61/00*** (2006.01)
***C07C 1/207*** (2006.01) ***C07C 11/04*** (2006.01)
***C07C 67/475*** (2006.01) ***C07C 69/74*** (2006.01)
***C07F 7/10*** (2006.01) ***C07F 9/6506*** (2006.01)
***C07F 15/00*** (2006.01) ***C07F 19/00*** (2006.01)

(52) Cooperative Patent Classification (CPC):
(C-Sets available)
**B01J 31/24; B01J 31/2273; B01J 31/2278; C07C 1/2074; C07C 67/333; C07F 15/0046; C07F 15/0073;** B01J 2231/54; B01J 2523/822; C07C 2531/24; C07C 2601/10; Y02P 20/52 (Cont.)

(86) International application number:
**PCT/JP2022/034764**

(87) International publication number:
**WO 2023/048084 (30.03.2023 Gazette 2023/13)**

(54) **ORGANIC METAL COMPLEX CATALYST FOR OLEFIN METATHESIS REACTION**

ORGANISCHER METALLKOMPLEXKATALYSATOR FÜR OLEFINMETATHESEREAKTION

COMPLEXE ORGANOMÉTALLIQUE CATALYTIQUE POUR RÉACTION DE MÉTATHÈSE D'OLÉFINES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.09.2021 JP 2021154173**

(43) Date of publication of application:
**31.07.2024 Bulletin 2024/31**

(73) Proprietors:
- **N.E. Chemcat Corporation
Tokyo 105-6124 (JP)**
- **National Institute of Advanced Industrial Science and Technology
Chiyoda-ku
Tokyo 100-8921 (JP)**

(72) Inventors:
- **CHOI, Junchul
Tsukuba-shi, Ibaraki 305-8560 (JP)**
- **FUKAYA, Norihisa
Tsukuba-shi, Ibaraki 305-8560 (JP)**
- **MATSUMOTO, Kazuhiro
Tsukuba-shi, Ibaraki 305-8560 (JP)**
- **MIZUSAKI, Tomoteru
Tokyo 105-6124 (JP)**
- **TAKAGI, Yukio
Tokyo 105-6124 (JP)**
- **SEKI, Yasuhiro
Tokyo 105-6124 (JP)**

(74) Representative: **Mewburn Ellis LLP
Aurora Building
Counterslip
Bristol BS1 6BX (GB)**

(56) References cited:
**EP-A1- 3 552 699**
**WO-A1-2018/105671**
**WO-A1-2021/177000**
**WO-A2-2011/069134**
**DE-T2- 60 004 817**
**JP-A- 2007 501 814**
**JP-A- 2016 509 006**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**C07C 1/2074, C07C 11/04;**
**C07C 67/333, C07C 69/74**

## Description

THECHINICAL FIELD

The present invention relates to an organic metal complex catalyst used in the olefin metathesis reaction. More specifically, the present invention relates to an organic metal complex catalyst having a ligand including a nitrogen-containing heterocyclic carbene structure and having ruthenium as a coordination center and used in the olefin metathesis reaction.

BACKGROUND ARTS

Olefin metathesis reactions such as ring-opening metathesis polymerization of olefin compounds, ring-closing metathesis reaction, cross-metathesis reaction of acyclic olefins, and metathesis polymerization of acyclic dienes are industrially useful reactions (for example, see Non-Patent Document 1).

As a catalyst used in the olefin metathesis reactions, there is known a Grubbs catalyst (first generation Grubbs catalyst) typically represented by benzylidene bis(tricyclohexylphosphine) dichlororuthenium represented by the following Formula (G1) (for example, see Non-Patent Document 2).

[Chem. 1]

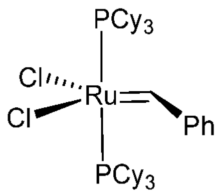

・・・(G 1)

Here, in the description, "Ph" represents a phenyl group, and "$PCy_3$" represents tricyclohexylphosphine.

Furthermore, a catalyst used in the olefin metathesis reactions, recently there has been attracted a ruthenium complex catalyst composed of a ligand including a structure of a nitrogen-containing heterocyclic carbene (N-Heterocyclic Carbene, hereinafter referred to as "NHC" as necessary) represented by the following Formula (G2) and a coordination center of ruthenium (hereinafter referred to as "NHC-Ru complex catalysts" as necessary) as a second-generation Grubbs catalysts (for example, see Non-Patent Documents 3 and 4).

[Chem. 2]

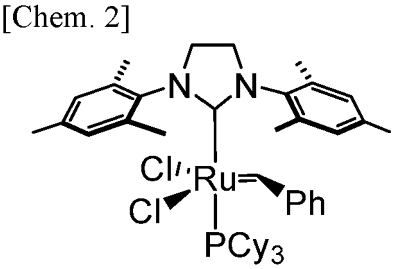

・・・(G 2)

It is known that this NHC-Ru complex catalyst has a high coordination ability to ruthenium due to the strong $\sigma$ donor property and weak $\pi$ acceptor property of NHC, and is stable in the complex state in air and water.

In particular, there are known that a ruthenium-carbene complex having a benzylidene carbene ligand and two tricyclohexylphosphine ligands, an NHC-Ru complex catalyst having 1,3-dimesitylimidazolidin-2-ylidene as a ligand, and an NHC-Ru complex catalyst having a 1,3-dimesitylimidazolin-2-ylidene as a ligand is to be a highly active catalyst for metathesis reactions (for example, see Patent Documents 1 to 3).

Further, as a catalyst that further improves the NHC-Ru complex catalyst of Formula (G2), Patent Document 4 discloses an organic metal complex catalyst such as a Pd complex catalyst having a 1,3-bis(2,6-isopropylphenyl)-2-imidazolidinylidene or a silyl-introduced 1,3-bis(2,6-isopropylphenyl)-2-imidazolidinylidene as a ligand and palladium as a coordination center (hereinafter referred to as "NHC-Pd complex catalyst" as necessary) as a catalyst used in the cross-coupling reactions.

Note that, the applicant of the patent application presents the following publications as publications in which the inventions known in the above-mentioned documents are described.

PRIOR ART DOCUMENT

Non-Patent Document

Non-Patent Document 1: Cesar A. Urbina-Blanco et al., J. Am. Chem. Soc. 2013, 135, 7073 - 7079.
Non-Patent Document 2: P. Schwab et al. : Angew. Chem., Int. Ed., 34, 2039 (1995).
Non-Patent Document 3: Jafarpour, L., Stevens, E. D., Nolan, S. P. J. Organomet. Chem. 2000, 606, 49-54.
Non-Patent Document 4: M. Scholl, et al. : Org. Lett., 1, 953 (1999).

Patent Document

Patent Document 1: JP Hei11-262667A
Patent Document 2: JP 2003-500412A
Patent Document 3: JP 2002-524250A
Patent Document 4: WO 2018-105672

SUMMARY OF THE INVENTION

PROBLEM TO BE SOLVED BY THE INVENTION

However, from the viewpoint of obtaining a high yield of the target product in the olefin metathesis reaction, the present inventors have found that even the catalysts of the above-mentioned prior art still have room for improvement.

The present invention was made in view of such technical circumstances, and an object of the present invention is to provide an organic metal complex catalyst that can obtain a higher yield of a target product than conventional catalysts in the olefin metathesis reaction.

MEANS TO SOLVE THE PROBLEM

As a result of intensive studies for solving the above-mentioned problem, the present inventors have found the construction of an organic metal complex catalyst having a structure represented by the following Formula (1) where a substituent "$-SiR^1R^2R^3$" (hereinafter referred to as a "silyl group" as necessary) containing a bonded silicon atom is bonded to the carbon atom at the 4th or 5th position (hereinafter referred to as "backbone carbon" as necessary) in the structure of NHC of the imidazole ring.

More specifically, the present invention consists of the following technical matters.

That is, the present invention provides an organic metal complex catalyst according to claim 1, which is used in the olefin metathesis reaction and comprises a chemical structure represented by the following Formula (1).

[Chem. 3]

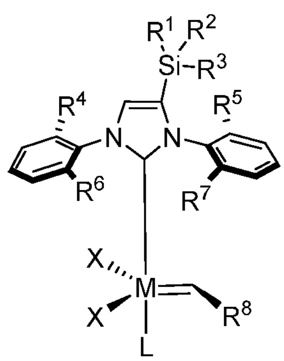

・・・(1)

Here, in the Formula (1), M is a coordination center and represents an Ru atom or an ion thereof.

Further, $R^1$, $R^2$ and $R^3$ may be the same or different, and each represents at least one substituent selected from

the group consisting of hydrogen atom, an alkyl group, an alkoxy group, an alkenyl group, an alkynyl group, and an aryl group.

Furthermore, $R^4$, $R^5$, $R^6$, and $R^7$ may be the same or different, and each represents at least one substituent selected from the group consisting of hydrogen atom, a halogen atom, an alkyl group, an alkoxy group, an alkenyl group, an alkynyl group, an aryl group, hydroxy group, a hydroxylate group, thiocarboxy group, dithiocarboxy group, sulfo group, sulfino group, oxycarbonyl group, carbamoyl group, hydradinocarbonyl group, amidino group, cyano group, isocyano group, cyanato group, isocyanato group, thiocyanato group, isothiocyanato group, formyl group, oxo group, thioformyl group, thioxo group, mercapto group, amino group, imino group, hydrazino group, allyloxy group, sulfide group, nitro group, and silyl group.

Further, in the Formula (1), X represents a halogen atom which is capable of coordinating to the coordination center M.

L represents a phosphorus ligand which is capable of coordinating to the coordination center M, and.

Furthermore, $R^8$ represents an alkyl group, an alkoxy group, an alkenyl group, an alkynyl group, or an aryl group having 3 to 20 carbon atoms.

The organic metal complex catalyst of the present invention having the above-mentioned structure can obtain the target product in a higher yield than the conventional catalysts (Grubbs catalyst, and the like) exemplified in the prior art documents mentioned above in the olefin metathesis reaction. Moreover, the organic metal complex catalyst of the present invention can relatively shorten the reaction time.

Although the detailed mechanism as to why the organic metal complex catalyst of the present invention can obtain the target product in a high yield has not been found, the present inventors speculate as follows.

That is, the present inventors think that the fact that the organic metal complex catalyst of the present invention has the structure in which the above-mentioned silyl group ($-SiR^1R^2R^3$) is bonded to the backbone carbon at the 4th or 5th position in the NHC structure contributes to improving the yield of the target product, but in contrast to the fact that the conventional catalyst is a ligand having a structure in which a hydrogen atom is bonded to the backbone carbon at the 4th or 5th position in the NHC structure of the imidazole ring {for example, a ligand having the NHC structure shown in Formula (P1) {(1,3-bis(2,6-diisopropylphenyl)imidazol-2-ylidene), hereinafter referred to as "IPr ligand"}.

[Chem. 4]

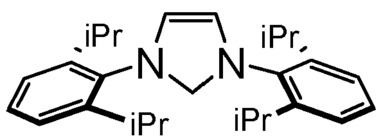

IPr

・・・(P 1)

Further, with respect to the ligand (the following Formula (2)) in which a silyl group is bonded to the backbone carbon at the 4th or 5th position in the NHC structure of the imidazole ring, the present inventors investigates the electron donating property to the central metal by measuring the TEP value (Tolman electronic paramater) [$cm^{-1}$] obtained from infrared spectroscopy.

Further, the present inventors also measured the TEP value with respect to the ligand (the following Formula (2-1)) having the structure in which hydrogen is bonded to the backbone carbon at the 4th or 5th position in the NHC structure of the imidazole ring.

Then, comparing the organic metal complex catalysts having the above two types of ligands, it was found that the organic metal complex catalyst having the ligand with higher electron donating ability to the central metal was also effective than the IPr ligand (Formula (P1)).

That is, in the organic metal complex catalyst for the olefin metathesis reaction of the present invention, $R^1$, $R^2$, $R^3$, $R^4$, $R^5$, $R^6$ and $R^7$ in the Formula (1) are arranged in combination, with respect to electron donating property of a ligand having a nitrogen-containing heterocyclic carbene structure represented by the following Formula (2) including these substituents to the coordination center M so that a TEP value (Tolman electronic paramater) [$cm^{-1}$] obtained from infrared spectroscopy is shifted to a lower wavenumber side in comparison with a TEP value [$cm^{-1}$] of a ligand represented by the following Formula (2-1).

[Chem. 5]

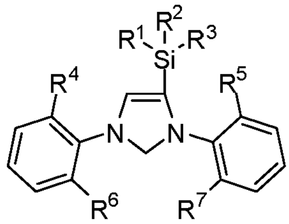

・・・(2)

[Chem. 6]

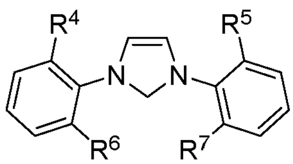

・・・(2−1)

Here, in the Formula (2), $R^1$, $R^2$, $R^3$, $R^4$, $R^5$, $R^6$ and $R^7$ represent the same substituents as $R^1$, $R^2$, $R^3$, $R^4$, $R^5$, $R^6$ and $R^7$ in the Formula (1).

Further, in the Formula (2-1), $R^4$, $R^5$, $R^6$ and $R^7$ represent the same substituents as $R^4$, $R^5$, $R^6$ and $R^7$ in the Formula (1).

Furthermore, with respect to the Rh carbonyl complex in which the moiety represented by $-MR^8X$ of the organic metal complex of the present invention was replaced with $-Rh(CO)_2Cl$, the present inventors measured the TEP value obtained by using the infrared absorption spectrum.

As a result, the present inventors found that among the ligands represented by Formula (2), the TEP value shifts to the lower wavenumber side than the IPr ligand (Formula (P1)), that is, the organic metal complex catalyst having the ligand with a higher electron donating property than the IPr ligand (Formula (P1)) gave a higher yield of the target product than the first generation Grubbs catalyst represented by the Formula (G1) (Comparative Example 2 described later), or the second generation Grubbs catalyst represented by the Formula (G2) (Comparative Example 1 described later).

Then, from these results, the present inventors consider that, when employing the structure that the NHC structure of the imidazole ring has the structure in which the silyl group ($-SiR^1R^2R^3$) is bonded to the backbone carbon at the 4th or 5th position, and the structure that the TEP value satisfies the conditions as described above, $M^0$ (zero valent), which is a catalytically active species during the catalytic reaction, will be stabilized, and the target product will be obtained in high yield.

Further, in the organic metal complex catalyst for olefin metathesis reaction of the present invention, it is that the TEP value [$cm^{-1}$] of the ligand having the nitrogen-containing heterocyclic carbene structure represented by the Formula (2) is a value determined from a stretching vibration frequency [$cm^{-1}$] of the carbonyl group obtained from the infrared absorption spectrum measured for the Rh carbonyl complex represented by the following Formula (1-1) where the moiety represented by $-MR^8X$ in the Formula (1) is substituted with $-Rh(CO)_2Cl$.

[Chem. 7]

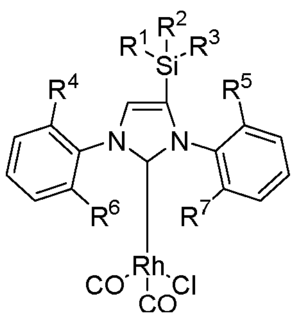

・・・(1−1)

In this case, the TEP value can be determined by the following Equation (E1).

[Eq. 1]

$$\mathrm{TEP}\ [\mathrm{cm}^{-1}] = \nu_{\mathrm{CO}}^{\mathrm{av/Ni}}\ [\mathrm{cm}^{-1}] = 0.8001\nu_{\mathrm{CO}}^{\mathrm{av/Rh}}\ [\mathrm{cm}^{-1}] + 420.0\ [\mathrm{cm}^{-1}] \quad \cdots (\mathrm{E1})$$

Here, in the Equation (E1), $\nu_{CO}^{av/Rh}$ represents the arithmetic average value of the stretching vibration frequency $[\mathrm{cm}^{-1}]$ of the carbonyl group obtained from the infrared absorption spectrum measured for the Rh carbonyl complex, and $\nu_{CO}^{av/Ni}$ represents the arithmetic average value [$cm^{-1}$] of the stretching vibration frequency of the carbonyl group of the Ni carbonyl complex (=TEP value [$cm^{-1}$]).

In the present invention, as the method for evaluating the electron donating property of the ligand containing the NHC structure of the organic metal complex catalyst for olefin metathesis reaction to the central metal by using the TEP value calculated according to the above-mentioned Equation (E1), the method described in the Non-Patent Document "T. Droge and F. Glorius, Angew. Chem. Int. Ed., 2010, 49, 6940" is adopted.

The TEP value (Tolman electronic paramater) is originally the stretching frequency of a carbonyl group obtained from the infrared absorption spectrum of a Ni carbonyl complex with Ni as the coordination center. However, the Ni carbonyl complex is highly toxic, making it difficult for an operator to measure the infrared absorption spectrum. Therefore, by using the stretching frequency of the carbonyl group obtained from the infrared absorption spectrum of the Rh carbonyl complex and the Equation (E1), it is possible for the operator to carry out the measurement work of the infrared absorption spectrum in an environment with improved safety.

Furthermore, from the viewpoint of more reliably obtaining the effects of the present invention, it is preferable that the organic metal complex catalyst for olefin metathesis reaction of the present invention has a structure represented by the following Formula (3).

[Chem. 8]

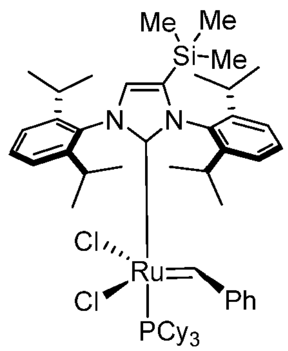

・・・(3)

Here, in the Formula (3), Me represents methyl group, Ph represents phenyl group, and $PCy_3$ represents tricyclohexylphosphine.

Further, from the viewpoint of more reliably obtaining the effects of the present invention, it is preferable that the organic metal complex catalyst of the present invention is used in a ring-closing metathesis reaction of a chain diolefin compound.

Furthermore, from the viewpoint of more reliably obtaining the effects of the present invention, it is preferable that the organic metal complex catalyst of the present invention is used in a ring-opening metathesis polymerization reaction of a cyclic olefin compound. The second generation Grubbs catalyst represented by the Formula (G2) is commercially available and is used in the ring-opening metathesis polymerization reactions of a cyclic olefin compound. The organic metal complex catalyst of the present invention has a structure in which a silyl group ($-SiR^1R^2R^3$) is further bonded to the backbone carbon at the 4th or 5th position of the IPr ligand, compared to the second generation Grubbs catalyst represented by the Formula (G2), and it is possible to similarly apply to the olefin metathesis reaction to which the second generation Grubbs catalyst is applied.

EFFECTS OF THE INVENTION

According to the present invention, the organic metal complex capable of obtaining a higher yield of a target product than conventional catalysts in the olefin metathesis reactions is provided.

BRIEF DESCRIPTION OF THE DRAWINGS

FIG. 1 is a graph showing a $^1$H NMR spectrum obtained for the organic metal complex catalyst for the olefin metathesis reactions of Example 1.
FIG. 2 is a graph showing a $^{29}$Si NMR spectrum obtained for the organic metal complex catalyst for the olefin metathesis reactions of Example 1.
FIG. 3 is a graph showing a $^{31}$P NMR spectrum obtained for the organic metal complex catalyst for the olefin metathesis reactions of Example 1.

MODE FOR CARRYING OUT THE INVENTION

Hereinafter, preferred embodiments of the present invention will be described in detail.

<Construction of organic metal complex catalyst for olefin metathesis reaction>

The organic metal complex catalyst for olefin metathesis reaction of the present embodiment is an organic metal complex catalyst used in the olefin metathesis reaction, preferably in the ring-closing metathesis reaction of the linear diolefin compounds and the ring-opening metathesis polymerization of the cyclic olefin compounds, and has the structure represented by the following Formula (1).

Further, the ligand of the organic metal complex catalyst for olefin metathesis reaction of the present embodiment has the structure of the nitrogen-containing heterocyclic carbene represented by the following Formula (2).

[Chem. 9]

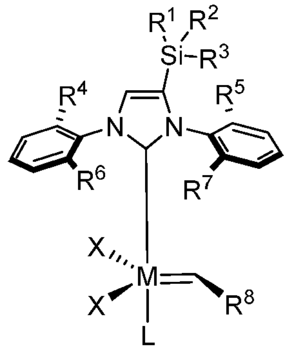

・・・(1)

Here, in Formula (1), M is a coordination center and represents an Ru atom or an ion thereof.

Further, $R^1$, $R^2$ and $R^3$ may be the same or different, and each represents at least one substituent selected from the group consisting of hydrogen atom, an alkyl group, an alkoxy group, an alkenyl group, an alkynyl group, and an aryl group.

Furthermore, $R^4$, $R^5$, $R^6$, and $R^7$ may be the same or different, and each represents at least one substituent selected from the group consisting of hydrogen atom, a halogen atom, an alkyl group, an alkoxy group, an alkenyl group, an alkynyl group, an aryl group, hydroxy group, a hydroxylate group, thiocarboxy group, dithiocarboxy group, sulfo group, sulfino group, oxycarbonyl group, carbamoyl group, hydradinocarbonyl group, amidino group, cyano group, isocyano group, cyanato group, isocyanato group, thiocyanato group, isothiocyanato group, formyl group, oxo group, thioformyl group, thioxo group, mercapto group, amino group, imino group, hydrazino group, allyloxy group, sulfide group, nitro group, and silyl group.

Further, in the Formula (1), X represents a halogen atom which is capable of coordinating to the coordination center M.

L represents a phosphorus ligand which is capable of coordinating to the coordination center M.

Furthermore, $R^8$ represents an alkyl group, an alkoxy group, an alkenyl group, an alkynyl group, or an aryl group having 3 to 20 carbon atoms.

Further, $R^1$, $R^2$, $R^3$, $R^4$, $R^5$, $R^6$ and $R^7$ in the Formula (1) are arranged in combination, with respect to electron donating property of a ligand having a nitrogen-containing heterocyclic carbene structure represented by the following Formula (2) including these substituents to the coordination center M so that a TEP value (Tolman electronic paramater) [cm-1] obtained from infrared spectroscopy is shifted to a lower wavenumber side in comparison with a TEP value [cm-1] of

a ligand represented by the following Formula (2-1).

[Chem. 10]

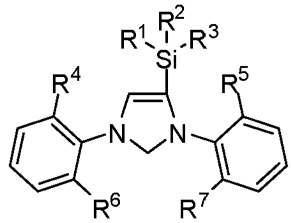

・・・(2)

[Chem. 11]

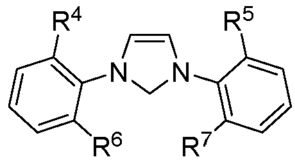

・・・(2－1)

Here, in the Formula (2), $R^1$, $R^2$, $R^3$, $R^4$, $R^5$, $R^6$ and $R^7$ represent the same substituents as $R^1$, $R^2$, $R^3$, $R^4$, $R^5$, $R^6$ and $R^7$ in the Formula (1).

Further, in the Formula (2-1), $R^4$, $R^5$, $R^6$ and $R^7$ represent the same substituents as $R^4$, $R^5$, $R^6$ and $R^7$ in the Formula (1).

According to the organic metal complex catalyst for olefin metathesis reaction of the present embodiment, which includes the ligand of the present embodiment having the above-mentioned configuration as the constituent material, it is possible to obtain a higher yield of the target product than the conventional Grubbs catalysts exemplified in Non-Patent Documents 1 to 4 mentioned above in the olefin metathesis reaction.

Although the detailed mechanism why the organic metal complex catalyst for olefin metathesis reaction of the present embodiment can obtain a high yield of the target product has not been solved, the present inventors think as follows.

That is, the present inventors think that, in contrast to that the conventional catalyst has the structure in which a hydrogen atom is bonded to the backbone carbon at the 4th or 5th position in the NHC structure of the imidazole ring (the structure of the IPr ligand (Formula (P1))), the organic metal complex catalyst of the present invention has the structure in which the above-mentioned silyl group ($-SiR^1R^2R^3$) is bonded to the backbone carbon at the 4th or 5th position in the NHC structure, which contributes to improving the yield of the target product. Furthermore, the present inventors think that the catalyst of the present embodiment has the structure that satisfies the above-mentioned TEP value condition, which further contributes to improving the yield of the target product.

With respect to the Rh carbonyl complex in which the moiety represented by $-MR^8X$ of the organic metal complex of the present embodiment was replaced with $-Rh(CO)_2Cl$, the present inventors measured the TEP value obtained by using the infrared absorption spectrum.

As a result, the present inventors found that among the ligands represented by Formula (2), the TEP value shifts to the lower wavenumber side than the IPr ligand (Formula (P1)), that is, the organic metal complex catalyst having the ligand having the NHC structure with a higher electron donating property than the IPr ligand (Formula (P1)) gave a higher yield of the target product than the conventional Grubbs catalysts represented by the Formulae (G1) and (G2).

Then, from these results, the present inventors consider that, when employing the structure that the NHC structure of the imidazole ring has the structure in which the silyl group ($-SiR^1R^2R^3$) is bonded to the backbone carbon at the 4th or 5th position, and the structure that the TEP value satisfies the conditions as described above, $M^0$ (zero valent), which is a catalytically active species during the catalytic reaction, will be stabilized, and the target product will be obtained in high yield (for example, see Example 1 later) .

Further, in the organic metal complex catalyst of the present embodiment, it is preferable that the TEP value [$cm^{-1}$] of the ligand having the nitrogen-containing heterocyclic carbene structure represented by the Formula (2) is a value determined from a stretching vibration frequency [$cm^{-1}$] of the carbonyl group obtained from the infrared absorption spectrum measured for the Rh carbonyl complex represented by the following Formula (1-1) where the moiety

represented by $-MR^8X$ in the Formula (1) is substituted with $-Rh(CO)_2Cl$.

[Chem. 12]

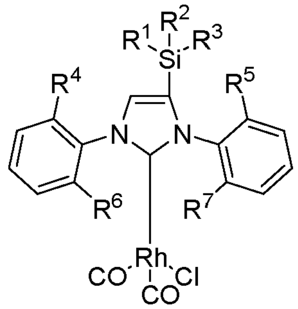

・・・(1－1)

In this case, the TEP value can be determined by the following Equation (E1).

[Eq. 2]

$$\mathrm{TEP}\ [\mathrm{cm}^{-1}] = \nu_{\mathrm{CO}}^{\mathrm{av/Ni}}\ [\mathrm{cm}^{-1}] = 0.8001\nu_{\mathrm{CO}}^{\mathrm{av/Rh}}\ [\mathrm{cm}^{-1}] + 420.0\ [\mathrm{cm}^{-1}] \quad \cdots (\mathrm{E1})$$

Here, in the Equation (E1), $\nu_{CO}^{av/Rh}$ represents the arithmetic average value of the stretching vibration frequency [$cm^{-1}$] of the carbonyl group obtained from the infrared absorption spectrum measured for the Rh carbonyl complex, and $\nu_{CO}^{av/Ni}$ represents the arithmetic average value [$cm^{-1}$] of the stretching vibration frequency of the carbonyl group of the Ni carbonyl complex (=TEP value [$cm^{-1}$] ).

In the present invention, as the method for evaluating the electron donating property of the ligand containing the NHC structure of the organic metal complex catalyst to the central metal by using the TEP value calculated according to the above-mentioned Equation (E1), the method described in the Non-Patent Document "T. Droge and F. Glorius, Angew. Chem. Int. Ed., 2010, 49, 6940" is adopted.

The TEP value (Tolman electronic paramater) is originally the stretching frequency of a carbonyl group obtained from the infrared absorption spectrum of a Ni carbonyl complex with Ni as the coordination center. However, the Ni carbonyl complex is highly toxic, making it difficult for an operator to measure the infrared absorption spectrum. Therefore, by using the stretching frequency of the carbonyl group obtained from the infrared absorption spectrum of the Rh carbonyl complex and the Equation (E1), it is possible for the operator to carry out the measurement work of the infrared absorption spectrum in an environment with improved safety.

Here, the coordination center M is a Ru atom or an ion thereof.

It is preferable that at least one of $R^1$, $R^2$ and $R^3$ is an alkyl group or an alkoxy group from the viewpoint of more reliably obtaining the effects of the present invention.

More preferably, it is an alkyl group or an alkoxy group having 1 to 3 carbon atoms.

It is preferable that at least one of $R^4$, $R^5$, $R^6$, and $R^7$ is an alkyl group having 1 to 3 carbon atoms from the viewpoint of more reliably obtaining the effects of the present invention.

It is preferable that X is Cl among halogen atoms from the viewpoint of more reliably obtaining the effects of the present invention and the ease of obtaining raw materials.

It is preferable that $R^8$ is a substituent having 3 to 10 carbon atoms that has a $\pi$ bond that can coordinate to the coordination center M from the viewpoint of more reliably obtaining the effects of the present invention, and more preferably a substituent having 3 to 9 carbon atoms that has a $\pi$ bond that can coordinate to the preferable coordination center Pd.

Furthermore, from the viewpoint of more reliably obtaining the effects of the present invention, it is preferable that the organic metal complex catalyst for olefin metathesis reaction of the present invention has a structure represented by the following Formula (3).

[Chem. 13]

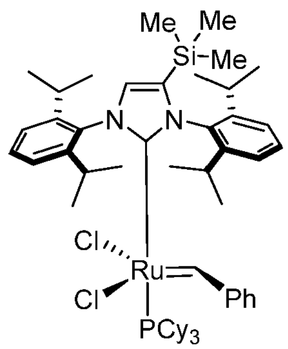

・・・(3)

Here, in the Formula (3), Me represents methyl group, Ph represents phenyl group, and $PCy_3$ represents tricyclohexylphosphine.

According to the present embodiment, it is possible to provide the organic metal complex catalyst that can obtain a higher yield of the target product than conventional catalysts in the olefin metathesis reaction.

Further, from the viewpoint of more reliably obtaining the effects of the present invention, it is preferable that the organic metal complex catalyst of the present invention is used in a ring-closing metathesis reaction of a chain diolefin compound. In the examples described below, it will be shown that the organic metal complex catalyst of the present invention has excellent catalytic performance for the ring-closing metathesis reaction of a chain diolefin compound.

Furthermore, from the viewpoint of more reliably obtaining the effects of the present invention, it is preferable that the organic metal complex catalyst of the present invention is used in a ring-opening metathesis polymerization reaction of a cyclic olefin compound. The second generation Grubbs catalyst represented by the Formula (G2) is commercially available and is used in the ring-opening metathesis polymerization reactions of a cyclic olefin compound. The organic metal complex catalyst of the present invention has a structure in which a silyl group ($-SiR^1R^2R^3$) is further bonded to the backbone carbon at the 4th or 5th position of the IPr ligand, compared to the second generation Grubbs catalyst represented by the Formula (G2), and it is easily possible to similarly apply to the olefin metathesis reaction to which the second generation Grubbs catalyst is applied.

<Preferred embodiment of method for producing organic metal complex catalyst>

The organic metal complex catalyst for olefin metathesis reaction of the present embodiment can be produced by, not particularly limited, combining and optimizing known methods for synthesizing ligands and methods for synthesizing complex catalysts.

The method for producing the organic metal complex catalyst for olefin metathesis reaction of the present embodiment includes:

a first step of synthesizing the ligand having the NHC structure represented by the Formula (2),
a second step of synthesizing a complex including the coordination center M, halogen atom X and the substituent $R^8$ in the Formula (1), and
a third step of synthesizing the organic metal complex catalyst for olefin metathesis reaction of the present embodiment by reacting the ligand having the NHC structure obtained in the first step and the complex obtained in the second step.

As the complex including the coordination center M, halogen atom X and the substituent $R^8$ in the Formula (1) used in the second step, the first generation Grubbs catalyst represented by the above Formula (G1), typically represented by benzylidene bis(tricyclohexylphosphine) dichlororuthenium represented by can be used.

Furthermore, the method for producing an organic metal complex catalyst for olefin metathesis reaction of the present embodiment further includes a fourth step of purifying the organic metal complex catalyst for olefin metathesis reaction of the present embodiment obtained after the third step. A known purification method can be adopted as the purification method in the fourth step. For example, a recrystallization method by using a predetermined solvent may be employed.

According to the method for producing the organic metal complex catalyst for olefin metathesis reaction of the present embodiment, it is possible to reliably produce the organic metal complex catalyst with the ligand for olefin

metathesis reaction and the organic metal complex catalyst that can obtain a higher yield of the target product than the conventional catalysts in the olefin metathesis reaction.

According to the production method of the present embodiment, it is possible to more easily and more reliably produce the organic metal complex catalyst with the ligand of the present embodiment for olefin metathesis reaction and the organic metal complex catalyst that can obtain a higher yield of the target product than the conventional catalysts in the olefin metathesis reaction.

According to the production method of the present embodiment, it is possible to easily produce the ligand of the present invention which has the structure in which the hydrogen bonded to the backbone carbon at the 4th or 5th position constituting the five-membered ring of the ligand having the NHC structure such as IPr is replaced with the silyl group, and has the structure that the TEP value satisfies the conditions as described above.

Conventionally, although the synthesis of a ligand having the NHC structure in which hydrogen on the backbone carbon is substituted with another substituent requires multiple synthesis steps, according to the production method of the present invention, it is possible to synthesize the ligand in which the silyl group is bonded to the backbone carbon at the 4th or 5th position through relatively few synthesis steps under relatively mild condition from the ligand in which the hydrogen is bonded to the backbone carbon at the 4th or 5th position such as IPr as the base. Moreover, in the production method of the present invention, various types of silyl groups can be introduced into the hydrogen moiety bonded to the backbone carbon at the 4th or 5th position by changing the silicon reagent used as the raw material.

For example, according to the production method of the present embodiment, as exemplified in the Formulas (C1) and (C2) below, it is possible to reduce the necessary synthesis steps from the IPr to the final product (the organic Ru complex catalyst having the ligand in which the hydrogen bonded to the backbone carbon of the ligand with the NHC structure is replaced with the silyl group) to relatively few three steps.

[Chem. 14]

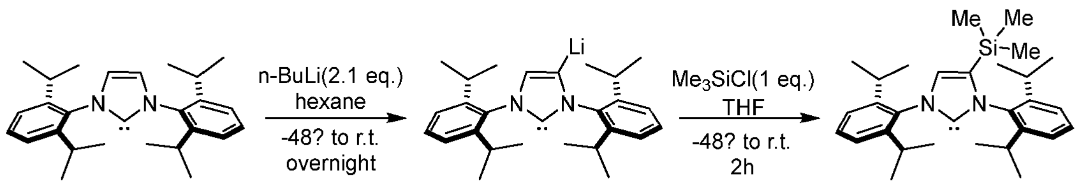


IPr Li-IPr $^{TMS}$IPr

・・・(C 1)

[Chem. 15]

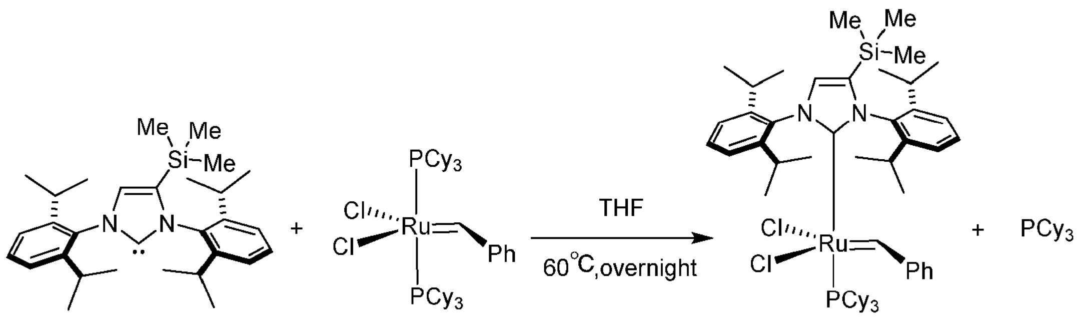


$^{TMS}$IPr First generation Grubbs catalyst $^{TMS}$IPr·Ru catalyst

・・・(C 2)

Here, in the Formula (C1) and Formula (C2), $^{n}BuLi$ represents $CH_3CH_2CH_2CH_2Li$, THF represents tetrahydrofuran, Me represents methyl group, Ph represents phenyl group, and $PCy_3$ represents tricyclohexylphosphine.

The synthesis method shown in the Formula (C1) is described, for example, in the Non-Patent Document: "Wang, Y et al., J. Am. Chem. Soc., 2010, 132, 14370".

An example is described in ".

The synthesis method of the Formula (C2) is a simple synthesis method discovered by the present inventors. For example, commercially available first generation Grubbs catalysts can be used.

EXAMPLE

In the following, the present invention will be explained in more detail with reference to Examples, but the present invention is not limited to the following Examples.

(Description of Analytical Apparatus)

The following apparatus was used for analysis when synthesizing the organic metal complex catalysts of Example 1, Comparative Example 1, and Comparative Example 2 described below.

[NMR Spectrum]

$^1$H NMR, $^{31}$P NMR, $^{29}$Si NMR spectrum, and $^{13}$C NMR were measured by using Bruker Biospin Avance 400 (400 MHz) available from Bruker. Dehydrated deuterated solvents were used in all measurements of the ligands. This is to prevent decomposition of the ligand.

(Example 1)

An organic metal complex catalyst {trade name "MTMS-RUA", available from N.E.CHEMCAT (hereinafter referred to as "$^{TMS}$IPrRu" as necessary)} was prepared. This $^{TMS}$IPrRu is the organic metal complex catalyst represented by the Formula (3).

The organic metal complex catalyst {$^{TMS}$IPrRu} of Example 1 was synthesized by the following procedures.

[Example 1 First Step-1] Synthesis of the ligand "IPr" having the NHC structure

2,6-Diisopropylaniline was used as a starting material, and the ligand "IPr" {1,3-bis(2,6-diisopropylphenyl) imidazol-2-ylidene} having the NHC structure represented by the above-mentioned Formula (P1) was synthesized.

Specifically, by referring the methods described in the academic papers (Tang, P., Wang, W., Ritter, T. J. Am. Chem. Soc. 2011, 133, 11482, and Pompeo, M., Froese, R. D. J., Hadei, N., Organ, M. G. Angew. Chem. Int. Ed. 2012, 51, 11354), synthesis was carried out through three steps shown by the following Reaction Schemes (R1) to (R3).

Identification was performed by using $^1$H NMR, and it was confirmed that the IPr and the intermediate products had been synthesized.

[Chem. 16]

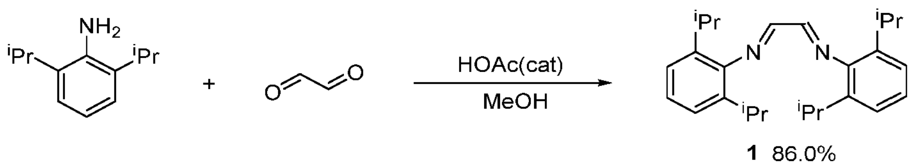


・・・(R 1)

In the Scheme (R1), MeOH represents methanol and HOAc represents acetic acid.

The procedure for synthesizing the intermediate product 1 in the Scheme (R1) is explained.

6.00 g (33.8 mmol) of 2,6-diisopropylaniline, 30 mL of methanol, and 0.31 mL (3.5 mol%) of acetic acid were added to a 50 mL recovery flask, and the mixture was heated to 50°C. Next, a mixed solution of 2.40g (0.5eq.) of glyoxal 40% aq. and 10 mL of methanol was added dropwise. As adding dropwise, the mixture was changed from a colorless and transparent solution to a yellow and transparent solution. After stirring at 50° C for 15 minutes, the mixture was returned to room temperature and further stirred for 11 hours. Upon cooling to room temperature, a yellow solid precipitated out. After the reaction was completed, filtration was performed by using a membrane filter, and the solid was washed with methanol. When washed, since a small amount of the target intermediate product 1 was dissolved in methanol, the filtrate was collected, the solvent was removed, and the resulting solid was washed again with a small amount of methanol and filtered. The yellow solids obtained in the first and second rounds were combined and dried.

The yield amount of the intermediate product 1 (yellow powder solid) in the Scheme (R1) was 5.49 g, and the yield was 86.0%.

[Chem. 17]

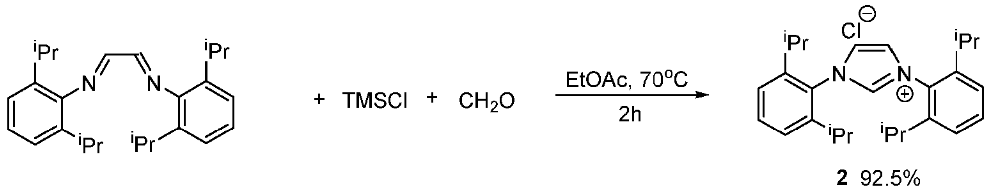


・・・(R 2)

In the Scheme (R2), TMSC1 represents chlorotrimethylsilane, and EtOAc represents ethyl acetate.

The procedure for synthesizing the intermediate product 2 in the Scheme (R2) is explained.

3.80 g (10.08 mmol) of (1E,2E)-1,2-bis(2,6-diisopropylphenylimino)ethane, 0.32 g (10.66 mmol) of paraformaldehyde, and 83 mL of ethyl acetate were added to a 500 mL recovery flask and the mixture was heated to 70°C. The mixed solution was in the state of a yellow slurry solution. Next, a mixed solution of 0.34 mL (10.66 mmol) of chlorotrimethylsilane and 8 mL of ethyl acetate was added dropwise over 20 minutes. Thereafter, the mixture was stirred at 70°C for 2 hours. The color of the solvent was changed from yellow to orange. After the reaction was completed, by immersing in an ice water and cooled to 0°C. After cooling, filtration was performed by a membrane filter, and the solid was washed with ethyl acetate. After vacuum drying, a pale pink powder solid was obtained.

The yield amount of the intermediate product 2 (white powder solid) in the Scheme (R2) was 3.96 g, and the yield was 92.5%.

[Chem. 18]

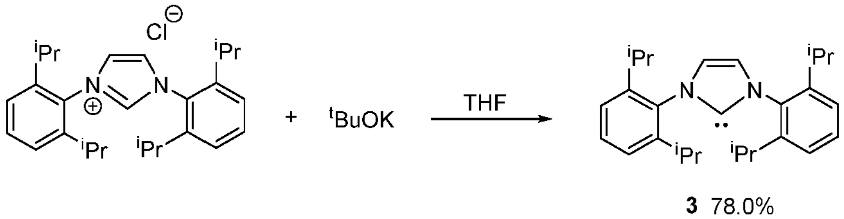


・・・(R 3)

In the Scheme (R3), $^tBuOK$ represents $(CH_3)_3COK$, and THF represents tetrahydrofuran.

The procedure for synthesizing product 3 "IPr" in the Scheme (R3) is explained.

Under an inert gas atmosphere, 0.43 g (1.01 mmol) of 1,3-bis(2,6-diisopropylphenyl)imidazolium chloride, 0.14 g (1.21 mmol) of $^tBuOK$ and 5 mL of dehydrated THF were added to 25 mL Schlenk and the mixture was stirred at room temperature for 3.5 hours. The white solution turned into a brown solution. After the completion of the reaction, the solvent was removed, 5 mL of dehydrated toluene was added, and the solid was dissolved by heating and stirring at 50°C. Thereafter, 5 mL of dehydrated hexane was added. In order to remove a salt (KCl) in the solution, celite filtration was performed in a glove box. A brown transparent solution was obtained. The solvent was removed and dried under vacuum to obtain a brown powder solid.

The yield amount of the product 3 "IPr" (brown powder solid) in the Scheme (R3) was 0.30 g, and the yield was 78.0%.

Identification was performed by using $^1H$ NMR, and it was confirmed that the IPr and the intermediate products (the intermediate product 1 in the Scheme (R1) and the intermediate product 2 in the Scheme (R2)) had been synthesized.

With respect to each of the ligands having the NHC structure represented by the Reaction Schemes (R1) to (R3), the $^1H$ NMR spectra were measured and confirmed. In the $^1H$ NMR spectrum measurement of the intermediate product 1 in the Scheme (R1), $CDCl_3$ was used as a deuterated solvent. In the $^1H$ NMR spectrum measurement of the intermediate product 2 in the Scheme (R2), $CD_3CN$ was used as a deuterated solvent. In the $^1H$ NMR spectrum measurement of the IPr represented by the product 3 in the Scheme (R3), $C_6D_6$ was used as a deuterated solvent.

The measurement results of the intermediate product 1 are shown below.

$^1H$ NMR ($CDCl_3$, 400 MHz): δ8.10 (s, 2H), 7.20-7.13 (m, 6H), 2.94 (m, 4H), 1.21 (d, 24H, J = 6.8 Hz)

The measurement results of the intermediate product 2 are shown below.

$^1H$ NMR ($CD_3CN$, 400 MHz): δ9.35 (s, 1H), 7.87 (s, 2H), 7.65 (t, 2H, J = 7.5 Hz), 7.47 (d, 4H, J = 7.7 Hz), 2.41 (m, 4H), 1.26 (d, 12H, J = 6.8 Hz), 1.20 (d, 12H, J = 6.8 Hz)

The measurement results of the product 3 "IPr" are shown below.

$^1H$ NMR ($C_6D_6$, 400 MHz): δ7.31-7.27 (m, 2H), 7.19-7.17 (m, 4H), 6.61 (s, 2H), 2.96 (m, 4H), 1.29 (d, 12H, J = 6.8 Hz), 1.18 (d, 12H, J = 7.0 Hz)

[Example 1 First Step-2] Synthesis of the ligand having trimethylsilyl group bonded to the 4th carbon in the NHC structure of the IPr

By using the ligand IPr obtained in the above-mentioned [First Step-1], the ligand having the NHC structure (ligand represented by the following Formula (3-1)) used in the organic metal complex of Example 1 represented by the Formula (3) was synthesized.

[Chem. 19]

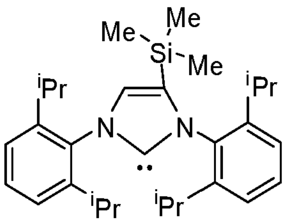

・・・(3－1)

Specifically, the method described in the academic paper (Wang, Y., Xie, Yaming., Abraham, M. Y., Wei, P., Schaefer III, H. F., Schleyer, P. R., Robinson, G. H. J. Am. Chem. Soc. 2010, 132, 14370) was modified, and through two steps represented by the following Reaction Scheme (R4), the ligand represented by the Formula (3-1) in which a trimethylsilyl group ($-SiMe_3$, hereinafter referred to as "TMS group" as necessary) is bonded to the 4th carbon of the NHC structure of the IPr (reactant 3) (hereinafter referred to as "$^{TMS}$IPr" as necessary) was synthesized.

[Chem. 20]

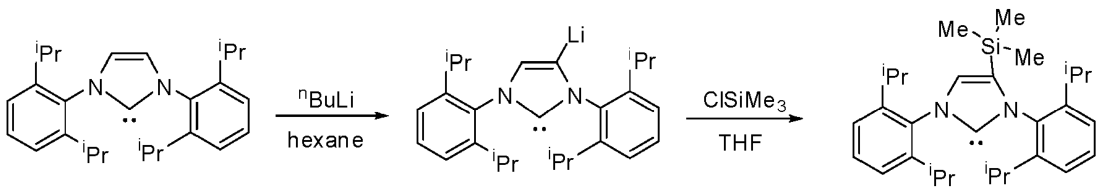


3 4 92.0% 5 98.9% ・・・(R 4)

In the Scheme (R4), $^nBuLi$ represents $CH_3CH_2CH_2CH_2Li$, and THF represents tetrahydrofuran.

The procedure for synthesizing the intermediate product 4 (Li-IPr) in the Scheme (R4) is explained.

At first, 10.79 g (27.62 mmol) of the IPr (reactant 3) and 100 mL of dehydrated hexane were added to a 300 mL recovery flask in a glove box, and the mixture was stirred at room temperature for 30 minutes. Next, $^nBuLi$ was slowly added dropwise to the obtained suspension, and the mixture was stirred and reacted overnight at room temperature. The solution changed from a light brown slurry to a yellow slurry. After the reaction was completed, filtration was performed by a membrane filter, and the solid was washed with dehydrated hexane. The obtained yellow powder solid {intermediate product 4 (lithiated product: Li-IPr) in the Scheme (R4)} was dried.

The yield amount of the intermediate product 4 (yellow powder solid) in the Scheme (R4) was 10.0 g, and the yield was 92.0%.

Next, the synthesis procedure of the product 5 ($^{TMS}$IPr) in the Scheme (R4) is explained.

At first, 0.78 g (1.98 mmol) of the intermediate product 4 (Li-IPr) and 25 mL of dehydrated THF were added to a 50 mL Schlenk in a glove box and dissolved. Next, 0.26 mL (2.04 mmol) of chlorotrimethylsilane ($ClSiMe_3$, hereinafter referred to as "ClTMS" as necessary) was slowly added dropwise, and the mixture was allowed to react for 25 minutes, and after the reaction was completed, the solvent was removed.

In the glove box, 10 mL of dehydrated toluene was added to the solid product to dissolve, and the resulting liquid was transferred to a centrifuge tube. The liquid in the centrifuge tube was centrifuged at 4000 rpm for 6 minutes at room temperature to separate the salt (LiCl). Next, the obtained filtrate was passed through a filter (available from Advantec, 0.2 μm) and separated into a 50 mL Schlenk. The solvent was then removed to obtain a yellow powder solid ($^{TMS}$IPr, that is the target ligand 5).

The yield amount of the product 5 "$^{TMS}$IPr" (yellow powder solid) in the Scheme (R4) was 0.901 g, and the yield was 98.9%.

Identification was performed by using $^1$H NMR, and it was confirmed that the lithiation of the hydrogen atom

bonded to the 4th carbon in the NHC structure of the IPr (reactant 3) progressed, and $^{TMS}$IPr (target ligand 5) was synthesized.

The measurement results for the product 5 "$^{TMS}$IPr" (target ligand 5) are shown below.

$^1$H NMR ($C_6D_6$, 400 MHz): δ=7.33-7.27 (m, 2H), 7.21-7.17 (m, 4H), 6.89 (s, 2H), 3.04 (m, 2H), 2.84 (m, 2H), 1.40 (d, 6H, J = 6.8 Hz), 1.28 (d, 12H, J =6.8 Hz, 6.9 Hz), 1.18 (d, 6H, J = 6.9 Hz), 0.05 ppm (s, 9H).

From the results of the $^1$H NMR, it was confirmed that due to the TMS group bonding to the 4th carbon in the NHC structure of the IPr (reactant 3), the proton peak derived from -CH of the $^i$Pr group became asymmetrical and was split into two.

Consumption of the mraw materials was also confirmed, and a peak derived from the methyl group of the TMS group was observed around 0 ppm. It was confirmed that $^{TMS}$IPr (target ligand 5) was synthesized since the chemical shift and integral value matched with the literatures. Furthermore, it was confirmed that the lithiation of the IPr (reactant 3) by $^n$BuLi was sufficiently progressing.

[Example 1 Second Step]

A commercially available the first generation Grubbs catalyst (trade name: Grubbs Catalyst M102, available from Aldrich) represented by the above-mentioned Formula (G1) was prepared.

[Example 1 Third Step] <Reaction of the ligand having the NHC structure obtained in the first step and the first generation Grubbs catalyst prepared in the second step>

The NHC structure obtained in the first step was The organic metal complex catalyst "$^{TMS}$IPrRu" of Example 1 was synthesized by carrying out the reaction represented by the following Reaction Scheme (R5) by using the ligand ($^{TMS}$IPr) and the first generation Grubbs catalyst prepared in the second step.

In this third step, the reaction conditions were independently investigated by the present inventors.

[Chem. 21]

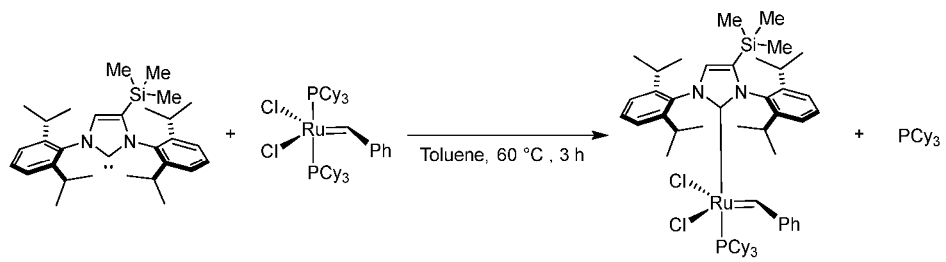


TMS-IPr G1 TMS-IPrRu

・・・(R 5)

The ligand ($^{TMS}$IPr) (40.0 mg, 0.0868 mmol) was placed in a 20 mL recovery flask in a glove box, and dehydrated toluene (5 mL) was added. To the solution, the first generation Grubbs catalyst: bis(tricyclohexylphosphine)benzylideneruthenium(IV) dichloride (G1) (59.5 mg, 0.0723 mmol) was added, and the mixture was stirred for 3 hours while heating to 60°C.

After the reaction was completed, toluene was distilled off and the product was vacuum dried for 30 minutes. The obtained solid was dissolved in dehydrated hexane, poured into a 13.5 mL vial, and recrystallized at -40°C. After the recrystallization, filtration was performed by using a membrane filter to obtain the target substance, reddish-purple solid $^{TMS}$IPrRu (6.2 mg, yield 99%).

[Example 1 Identification]

The identification of the $^{TMS}$IPrRu was confirmed by $^1$H NMR, $^{31}$P NMR, and $^{29}$Si NMR. $C_6D_6$ was used as a deuterated solvent in these three types of NMR measurements.

FIG. 1 shows a $^1$H NMR spectrum obtained for the organic metal complex catalyst {$^{TMS}$IPrRu} of Example 1. FIG. 2 shows the $^{31}$P NMR spectrum obtained for the organic metal complex catalyst {$^{TMS}$IPrRu} of Example 1. FIG. 3 shows the $^{29}$Si NMR spectrum obtained for the organic metal complex catalyst {$^{TMS}$IPrRu} of Example 1.

$^{1}H$ NMR (400MHz, $C_6D_6$): See FIG. 1.
$^{31}P$ NMR (162.1 MHz, $C_6D_6$): $\delta$(ppm) = 28.1
$^{29}Si$ NMR (119.2 MHz, $C_6D_6$): $\delta$(ppm) = -8. 13

From the results shown in FIG. 1, FIG. 2, and FIG. 3, it was determined that the target $^{TMS}$IPrRu could be synthesized.

Note that, in FIG. 2, the presence of a small amount of the first generation Grubbs catalyst represented by the Formula (G1) and the second generation Grubbs catalyst represented by the Formula (G2) was confirmed as impurities. It is thought that the first generation Grubbs catalyst remained in the reaction system as an unreacted product. Furthermore, it is thought that the second generation Grubbs catalyst is one in which the silyl group of $^{TMS}$IPr is removed during the reaction. Since $^{31}P$ NMR has high sensitivity, it is thought that these impurities were observed.

(Comparative Example 1)

The second generation Grubbs catalyst represented by the Formula (G2) was synthesized by the method described in Non-Patent Document 3.

(Comparative Example 2)

The commercially available first generation Grubbs catalyst represented by the Formula (G1) (available from Aldrich, trade name: "Grubbs Catalyst M102") was prepared.

(Example 1-Rh)

The organic metal complex catalyst (trade name "NTMS-RHA", available from N.E. CHEMCAT) was prepared. The Example 1-Rh is a catalyst having a structure in which the $-Ru(CH_2Ph)PCy_3Cl_2$ moiety of the organic metal complex catalyst of the above-mentioned Example 1 was replaced with $-Rh(CO)_2Cl$.

[Example 1-Rh First Step]

At first, the same synthesis procedure and analysis as in Example 1 were performed to synthesize a ligand having the NHC structure represented by the above-mentioned Formula (3-1).

[Example 1-Rh Second Step]

Next, a commercially available $[Rh(CO)_2Cl]_2$ available from Aldrich was prepared as a $\pi$ allyl Rh complex serving as an Rh source.

[Example 1-Rh Third Step]

Next, the reaction represented by the following Reaction Scheme (R6) was carried out by using the ligand having the NHC structure represented by the Formula (3-1) obtained in the first step and the $\pi$ allyl Rh complex prepared in the second step to synthesize the organic metal complex catalyst of Example 1-Rh.

[Chem. 22]

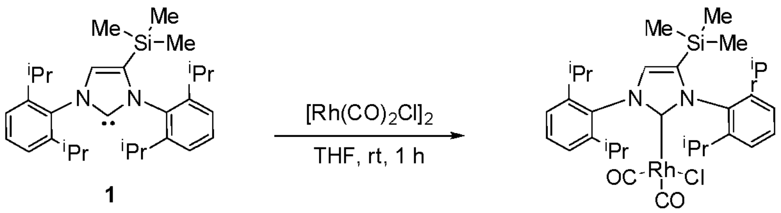


1

・・・(R 6)

[Example 1-Rh Identification]

The identification of the product of the Scheme (R11), that is, the organic metal complex catalyst of Example 1-Rh (trade name "NTMS-RHA", available from N.E. CHEMCAT) was confirmed by using $^{1}H$ NMR, $^{13}C$ NMR, $^{29}Si$ NMR,

MALDI-TOF-MS, and elemental analysis.

(Comparative Example 1-Rh)

The organic metal complex catalyst in which the $-Ru(CH_2Ph)PCy_3Cl_2$ moiety of the second generation Grubbs catalyst represented by the above-mentioned Formula (G2) was replaced with $-Rh(CO)_2Cl$ (hereinafter referred to as "IPrRh" as necessary) was prepared.

[Comparative Example 1-Rh First Step]

At first, the same synthesis procedure and analysis as in the first step-1 of Example 1 were performed to synthesize the ligand IPr having the NHC structure represented by the above-mentioned Formula (P1).

[Comparative Example 1-Rh Second Step]

Next, the commercially available $[Rh(CO)_2Cl]_2$ available from Aldrich was prepared as a $\pi$ allyl Rh complex serving as a Rh source.

[Comparative Example 1-Rh Third Step]

Next, the reaction represented by the following Reaction Scheme (R7) was carried out by using the ligand IPr having the NHC structure represented by the Formula (P1) obtained in the first step and the $\pi$ allyl Rh complex prepared in the second step to synthesize the organic metal complex catalyst IPrRh of Comparative Example 2-Rh.

[Chem. 23]

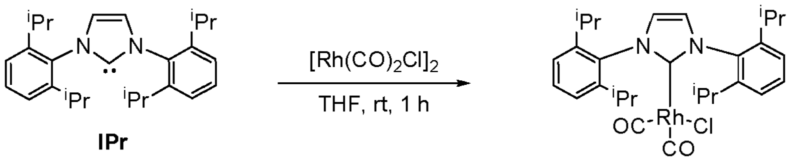


IPr

・・・(R 7)

[Comparative Example 1-Rh Forth Step]

Purification of the organic metal complex catalyst obtained after the third step

After the third step, recrystallization treatment was performed by using hexane or the like for the solid containing the product IPrRh of the Scheme (R7) to purify.

[Comparative Example 1-Rh Identification]

The identification of the product of the Scheme (R7), that is, the organic metal complex catalyst IPrRh of Comparative Example 1-Rh was confirmed by using $^{1}H$ NMR, $^{13}C$ NMR, $^{29}Si$ NMR, MALDI-TOF-MS, and elemental analysis.

<IR measurement of Example 1-Rh and Comparative Example 1-Rh>

Infrared absorption spectra were measured for the organic metal complex catalysts of Example 1-Rh and Comparative Example 1-Rh. Then, by using the arithmetic average value of the stretching vibration frequency [$cm^{-1}$] of the carbonyl group obtained from each infrared absorption spectrum, the TEP value [$cm^{-1}$] of the organic metal complex catalyst in which the coordination center was changed from Rh to Ni was determined according to the following Equation (E1) described above.

[Eq. 3]

$$\mathrm{TEP}\ [\mathrm{cm}^{-1}] = \nu_{\mathrm{CO}}^{\mathrm{av/Ni}}\ [\mathrm{cm}^{-1}] = 0.8001\nu_{\mathrm{CO}}^{\mathrm{av/Rh}}\ [\mathrm{cm}^{-1}] + 420.0\ [\mathrm{cm}^{-1}] \quad \cdots (\mathrm{E1})$$

Table 1 shows the TEP values determined for each organic metal complex catalyst.

[Table 1]

| | Trade name or abbreviation | Arithmetic average value of stretching vibration frequency of carbonyl group of Rh complex | Stretching vibration frequency of carbonyl group of Ni complex calculated from Equation E1 |
|---|---|---|---|
| | | $\nu_{CO}{}^{av/Rh}$ / $cm^{-1}$ | TEP / $cm^{-1}$ a |
| Ex 1-Rh | MTEMS-RHA | 2024.9 | 2040.1 (-3.5) |
| Com. Ex 1-Rh | IPrRh | 2029.2 | 2043.6 |
| *a The number in the parentheses indicates the difference between the TEP value of Comparative Example 1-Rh and the TEP value of each organic metal catalyst. | | | |

As is clear from the results shown in Table 1, it was confirmed that the TEP value of the organic metal complex catalyst of Example 1-Rh was shifted to the lower wavenumber side than the TEP value of Comparative Example 1-Rh. That is, it was found that the organic metal complex catalyst of Example 1-Rh had the ligand having the NHC structure with higher electron donating property than the IPr ligand (Formula (P1)) of Comparative Example 1-Rh.
From this fact, it was found that the organic metal complex catalyst of Example 1 in which the coordination center was substituted from Rh to Ru also has the ligand having the NHC structure with higher electron donating property than the IPr ligand (Formula (P1)) of Comparative Example 1.

<Catalytic activity evaluation by olefin metathesis reaction>

By using the organic metal complex catalysts of Example 1, Comparative Example 1 and Comparative Example 2, the olefin metathesis reaction (chain diolefin compound ring-closing metathesis reaction) represented by the Reaction Scheme (R8) was performed.

[Chem. 24]

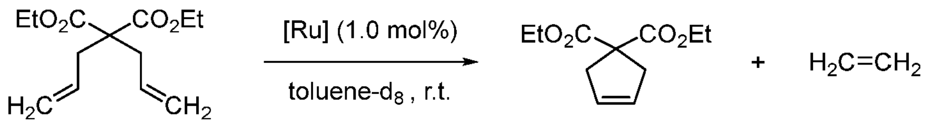


・・・(R 8)

$^{TMS}$IPrRu (8.1 mg, 8.06 μmol) of Example 1 was placed in a 4 cc vial, and toluene-$d_8$ (0.25 mL) was added to prepare a $^{TMS}$IPrRu/toluene-$d_8$ solution (16.0 μM).
In a glove box, diethyl diallymalonate (9.2 mg, 0.080 mmol) and an internal standard substance (1,3,5-trimethylbenzene, 9.6 mg, 0.080 mmol) were placed in a sealable NMR sample tube, and toluene-ds (0.4mL) was added as a solvent.
Next, the $^{TMS}$IPrRu/toluene-$d_8$ solution (50 μL, $^{TMS}$IPrRu: 0.80 μmol, 1.0 mol%) was added to this mixed solution, and the ring-closing metathesis reaction represented by the Reaction Scheme (R8) was performed at room temperature. The yield of the target cyclopentene compound was determined by $^1$H NMR measurement 15 minutes after the start of the reaction. The results are shown in Table 2.
With respect to the organic metal complex catalyst of Comparative Example 1 (second generation Grubbs catalyst represented by the Formula (G2)), the ring-closing metathesis reaction represented by the Reaction Scheme (R8) was carried out at room temperature under the same conditions and procedures as in the reaction evaluation test described above. The yield of the target cyclopentene compound was determined by $^1$H NMR measurement 15 minutes after the start of the reaction. The results are shown in Table 2.
With respect to the organic metal complex catalyst of Comparative Example 2 (first generation Grubbs catalyst represented by the Formula (G1)), the ring-closing metathesis reaction represented by the Reaction Scheme (R8) was carried out at room temperature under the same conditions and procedures as in the reaction evaluation test described above. The yield of the target cyclopentene compound was determined by $^1$H NMR measurement 15 minutes after the start of the reaction. The results are shown in Table 2.

[Table 2]

| | Catalyst | NMR yield 15 min after the start |
|---|---|---|
| Ex 1 | TMSIprRu(MTMS-RUA | 90% |
| Com. Ex 1 | IPrRu | 49% |
| Com. Ex 2 | G1 | 46% |

From the results shown in Table 2, compared to the organic metal complex catalysts of Comparative Example 1 and Comparative Example 2, which are conventional Grubbs catalysts, when the organic metal complex catalyst of Example 1, which satisfies the structure of the present invention, is used, it has become clear that the target product can be obtained in a very high yield for the olefin metathesis reactions (ring-closing metathesis reactions of chain diolefin compound).

INDUSTRIAL APPLICABILITY

The catalyst of the present invention can provide a higher yield of the target product than conventional catalysts in the olefin metathesis reactions. Therefore, the present invention contributes to the development of mass production technology in the fields of medicines, agricultural chemicals, and electronic materials in which the olefin metathesis reactions can be used to synthesize target products (e.g., aromatic amines).

EXPLANATION OF SYMBOLS

| | |
|---|---|
| IPr | 1,3-bis(2,6-diisopropylphenyl)imidazol-2-ylidene |
| NHC | Nitrogen-containing heterocyclic carbene (N-Heterocyclic Carbene) |
| TMS | Trimethylsilyl group |

**Claims**

1. An organic metal complex catalyst for olefin metathesis reaction, which is used in the olefin metathesis reaction and comprises a chemical structure represented by the following Formula (1):

[Chem. 1]

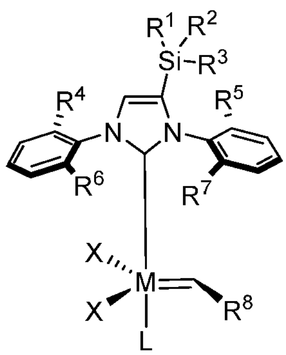

・・・(1)

in the Formula (1),

M is a coordination center and represents an Ru atom or an ion thereof,
$R^1$, $R^2$ and $R^3$ may be the same or different, and each represents at least one substituent selected from the group consisting of hydrogen atom, an alkyl group, an alkoxy group, an alkenyl group, an alkynyl group, and an aryl group,
$R^4$, $R^5$, $R^6$, and $R^7$ may be the same or different, and each represents at least one substituent selected from the group consisting of hydrogen atom, a halogen atom, an alkyl group, an alkoxy group, an alkenyl group, an alkynyl group, an aryl group, hydroxy group, a hydroxylate group, thiocarboxy group, dithiocarboxy group, sulfo group,

sulfino group, oxycarbonyl group, carbamoyl group, hydradinocarbonyl group, amidino group, cyano group, isocyano group, cyanato group, isocyanato group, thiocyanato group, isothiocyanato group, formyl group, oxo group, thioformyl group, thioxo group, mercapto group, amino group, imino group, hydrazino group, allyloxy group, sulfide group, nitro group, and silyl group,
X represents a halogen atom which is capable of coordinating to the coordination center M,
L represents a phosphorus ligand which is capable of coordinating to the coordination center M, and
$R^8$ represents an alkyl group, an alkoxy group, an alkenyl group, an alkynyl group, or an aryl group having 3 to 20 carbon atoms.
wherein $R^1$, $R^2$, $R^3$, $R^4$, $R^5$, $R^6$ and $R^7$ in the Formula (1) are arranged in combination, with respect to electron donating property of a ligand having a nitrogen-containing heterocyclic carbene structure represented by the following Formula (2) including these substituents to the coordination center M so that a TEP value (Tolman electronic paramater) [$cm^{-1}$] obtained from infrared spectroscopy is shifted to a lower wavenumber side in comparison with a TEP value [$cm^{-1}$] of a ligand represented by the following Formula (2-1).

[Chem. 2]

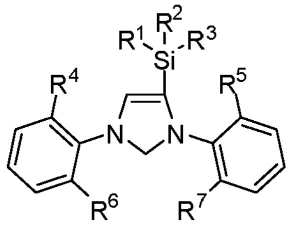

・・・(2)

[Chem. 3]

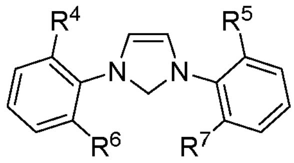

・・・(2－1)

In the Formula (2), $R^1$, $R^2$, $R^3$, $R^4$, $R^5$, $R^6$ and $R^7$ represent the same substituents as $R^1$, $R^2$, $R^3$, $R^4$, $R^5$, $R^6$ and $R^7$ in the Formula (1), in the Formula (2-1), $R^4$, $R^5$, $R^6$ and $R^7$ represent the same substituents as $R^4$, $R^5$, $R^6$ and $R^7$ in the Formula (1).

**2.** The organic metal complex catalyst for olefin metathesis reaction according to claim 1, wherein the TEP value of the ligand having the nitrogen-containing heterocyclic carbene structure represented by the Formula (2) is a value determined from a stretching vibration frequency of the carbonyl group obtained from the infrared absorption spectrum measured for the Rh carbonyl complex represented by the following Formula (1-1) where the moiety represented by $-MR^8X$ in the Formula (1) is substituted with $-Rh(CO)_2Cl$.

[Chem. 4]

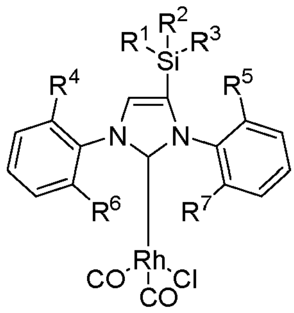

・・・(1－1)

3. The organic metal complex catalyst for olefin metathesis reaction according to claim 1 or 2, which has a chemical structure represented by the following Formula (3).

[Chem 5]

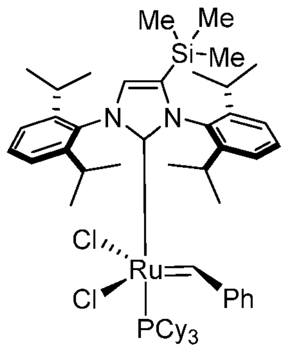

・・・(3)

in the Formula (3), Me represents methyl group, Ph represents phenyl group, and $PCy_3$ represents tricyclohexylphosphine.

4. Use of the organic metal complex catalyst according to claim 1 for ring-closing metathesis reactions of chain diolefin compounds.

5. Use of the organic metal complex catalyst according to claim 1 for ring-opening metathesis polymerization reactions of cyclic olefin compounds.

## Patentansprüche

1. Organischer Metallkomplex-Katalysator für eine Olefinmetathese-Reaktion, der in der Olefinmetathese-Reaktion verwendet wird und eine chemische Struktur umfasst, die durch die folgende Formel (1) dargestellt ist:

[Chem. 1]

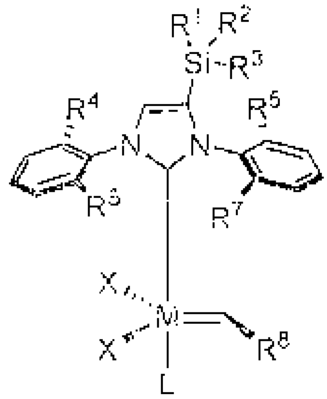

· · · (1)

wobei in der Formel (1):

M ein Koordinationszentrum ist und für ein Ru-Atom oder ein Ion davon steht,
$R^1$, $R^2$ und $R^3$ gleich oder unterschiedlich sein können und jeweils für zumindest einen Substituenten stehen, der aus der aus einem Wasserstoffatom, einer Alkylgruppe, einer Alkoxygruppe, einer Alkenylgruppe, einer Alkinylgruppe und einer Arylgruppe bestehenden Gruppe ausgewählt ist,
$R^4$, $R^5$, $R^6$ und $R^7$ gleich oder unterschiedlich sein können und jeweils für zumindest einen Substituenten stehen, der aus der aus einem Wasserstoffatom, einem Halogenatom, einer Alkylgruppe, einer Alkoxygruppe, einer Alkenylgruppe, einer Alkinylgruppe, einer Arylgruppe, einer Hydroxygruppe, einer Hydroxylatgruppe, einer Thiocarboxygruppe, einer Dithiocarboxygruppe, einer Sulfogruppe, einer Sulfinogruppe, einer Oxycarbonylgruppe, einer Carbamoylgruppe, einer Hydrazinocarbonylgruppe, einer Amidinogruppe, einer Cyanogruppe, einer Isocyanogruppe, einer Cyanatogruppe, einer Isocyanatogruppe, einer Thiocyanatogruppe, einer Isothiocyanatogruppe, einer Formylgruppe, einer Oxogruppe, einer Thioformylgruppe, einer Thioxogruppe, einer Mercaptogruppe, einer Aminogruppe, einer Iminogruppe, einer Hydrazinogruppe, einer Allyloxygruppe, einer Sulfidgruppe, einer Nitrogruppe und einer Silylgruppe bestehenden Gruppe ausgewählt ist,
X für ein Halogenatom steht, das dazu in der Lage ist, an das Koordinationszentrum M zu koordinieren,
L für einen Phosphor-Liganden steht, der dazu in der Lage ist, an das Koordinationszentrum M zu koordinieren, und
$R^8$ für eine Alkylgruppe, eine Alkoxygruppe, eine Alkenylgruppe, eine Alkinylgruppe oder eine Arylgruppe mit 3 bis 20 Kohlenstoffatomen steht,
worin $R^1$, $R^2$, $R^3$, $R^4$, $R^5$, $R^6$ und $R^7$ in Formel (1) im Hinblick auf die Elektronendonator-Eigenschaft eines Liganden, der eine stickstoffhältige heterozyklische Carben-Struktur aufweist, die durch die folgende Formel (2), die diese Substituenten umfasst, dargestellt ist, in Kombination so am Koordinatinszentrum M angeordnet sind, dass der durch Infrarot-Spektroskopie erhaltene Wert für den TEP (Tolmans elektronischen Parameter) [$cm^{-1}$] im Vergleich mit dem TEP-Wert [$cm^{-1}$] eines Liganden, der durch die folgende Formel (2-1) dargestellt ist, zu niedrigeren Wellenzahlen hin verschoben ist:

[Chem. 2]

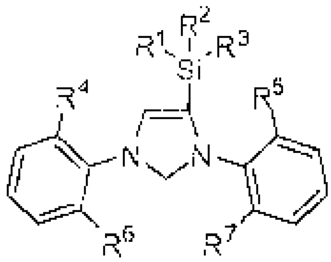

· · · (2)

[Chem. 3]

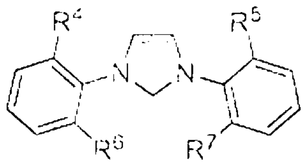

・・・(2-1)

wobei in der Formel (2) $R^1$, $R^2$, $R^3$, $R^4$, $R^5$, $R^6$ und $R^7$ für dieselben Substituenten wie $R^1$, $R^2$, $R^3$, $R^4$, $R^5$, $R^6$ und $R^7$ in Formel (1) stehen und wobei in der Formel (2-1) $R^4$, $R^5$, $R^6$ und $R^7$ für dieselben Substituenten wie $R^4$, $R^5$, $R^6$ und $R^7$ in Formel (1) stehen.

**2.** Organischer Metallkomplex-Katalysator für eine Olefinmetathese-Reaktion nach Anspruch 1, wobei der TEP-Wert des Liganden, der eine stickstoffhältige heterozyklische Carben-Struktur aufweist, die durch die Formel (2) dargestellt ist, jener Wert ist, der aus der Streckschwingungs-Frequenz der Carbonylgruppe bestimmt wird, die aus einem Infrarot-Absorptionsspektrum erhalten wird, das für einen Rh-Carbonyl-Komplex gemessen wird, der durch die folgende Formel (1-1) dargestellt ist, worin die in Formel (1) als $-MR^8X$ dargestellte Gruppierung durch $-Rh(CO)_2Cl$ ersetzt ist:

[Chem. 4]

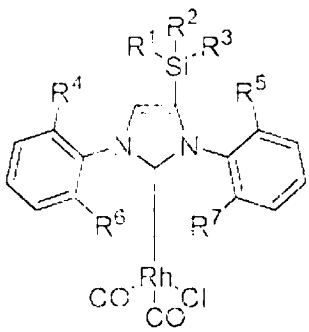

・・・(1-1)

**3.** Organischer Metallkomplex-Katalysator für eine Olefinmetathese-Reaktion nach Anspruch 1 oder 2, der eine chemische Struktur aufweist, die durch die folgende Formel (3) dargestellt ist:

[Chem. 5]

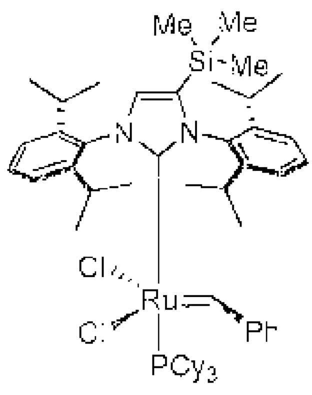

・・・(3)

wobei in Formel (3) Me für eine Methylgruppe steht, Ph für eine Phenylgruppe steht und $PCy_3$ für Tricyclohexylphosphin steht.

4. Verwendung eines organischen Metallkomplex-Katalysators nach Anspruch 1 für Ringschlussmetathese-Reaktionen von kettenförmigen Diolefin-Verbindungen.

5. Verwendung eines organischen Metallkomplex-Katalysators nach Anspruch 1 für Ringöffnungsmethatese-Polymerisationsreaktionen von zyklischen Olefin-Verbindungen.

## Revendications

1. Complexe organométallique catalytique pour une réaction de métathèse d'oléfines, qui est utilisé dans la réaction de métathèse d'oléfines et comprend une structure chimique représentée par la formule (1) suivante :

[Chem. 1]

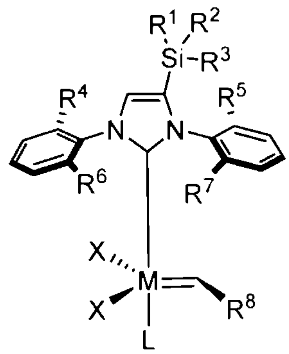

・・・(1)

dans la formule (1),

M est un centre de coordination et représente un atome de Ru ou un ion de celui-ci,
$R^1$, $R^2$ et $R^3$ peuvent être identiques ou différents, et chacun représente au moins un substituant choisi dans le groupe constitué par un atome d'hydrogène, un groupe alkyle, un groupe alcoxy, un groupe alcényle, un groupe alcynyle et un groupe aryle,
$R^4$, $R^5$, $R^6$, et $R^7$ peuvent être identiques ou différents, et chacun représente au moins un substituant choisi dans le groupe constitué par un atome d'hydrogène, un atome d'halogène, un groupe alkyle, un groupe alcoxy, un groupe alcényle, un groupe alcynyle, un groupe aryle, un groupe hydroxy, un groupe hydroxylate, un groupe thiocarboxy, un groupe dithiocarboxy, un groupe sulfo, un groupe sulfino, un groupe oxycarbonyle, un groupe carbamoyle, un groupe hydradinocarbonyle, un groupe amidino, un groupe cyano, un groupe isocyano, un groupe cyanato, un groupe isocyanato, un groupe thiocyanato, un groupe isothiocyanato, un groupe formyle, un groupe oxo, un groupe thioformyle, un groupe thioxo, un groupe mercapto, un groupe amino, un groupe imino, un groupe hydrazino, un groupe allyloxy, un groupe sulfure, un groupe nitro et un groupe silyle,
X représente un atome d'halogène qui est capable de se coordonner au centre de coordination M,
L représente un ligand phosphoré qui est capable de se coordonner au centre de coordination M, et
$R^8$ représente un groupe alkyle, un groupe alcoxy, un groupe alcényle, un groupe alcynyle ou un groupe aryle présentant de 3 à 20 atomes de carbone,
dans lequel $R^1$, $R^2$, $R^3$, $R^4$, $R^5$, $R^6$ et $R^7$ dans la formule (1) sont agencés en combinaison, par rapport à la propriété de don d'électrons d'un ligand présentant une structure de carbène hétérocyclique contenant de l'azote représenté par la formule (2) suivante incluant ces substituants au centre de coordination M de sorte qu'une valeur de TEP (paramètre électronique de Tolman) [$cm^{-1}$] obtenue par spectroscopie infrarouge est décalée vers un côté de nombre d'onde inférieur par comparaison avec une valeur de TEP [$cm^{-1}$] d'un ligand représenté par la formule (2-1) suivante

[Chem. 2]

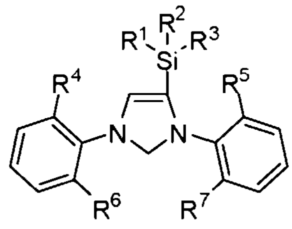

・・・(2)

[Chem. 3]

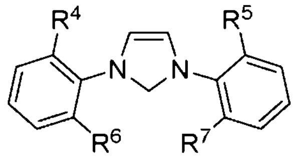

・・・(2 1)

dans la formule (2), $R^1$, $R^2$, $R^3$, $R^4$, $R^5$, $R^6$ et $R^7$ représentent les mêmes substituants que $R^1$, $R^2$, $R^3$, $R^4$, $R^5$, $R^6$ et $R^7$ dans la formule (1), dans la formule (2-1), $R^4$, $R^5$, $R^6$ et $R^7$ représentent les mêmes substituants que $R^4$, $R^5$, $R^6$ et $R^7$ dans la formule (1).

**2.** Complexe organométallique catalytique pour une réaction de métathèses d'oléfines selon la revendication 1, dans lequel la valeur de TEP du ligand présentant la structure de carbène hétérocyclique contenant de l'azote représenté par la formule (2) est une valeur déterminée à partir d'une fréquence de vibration d'étirement du groupe carbonyle obtenue à partir du spectre d'absorption infrarouge mesuré pour le complexe de carbonyle Rh représenté par la formule (1-1) suivante où la fraction représentée par $-MR^8X$ dans la formule (1) est substituée par $-Rh(CO)_2Cl$.

[Chem. 4]

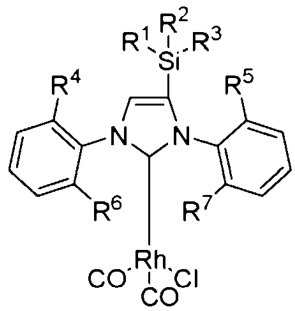

・・・(1－1)

**3.** Complexe organométallique catalytique pour une réaction de métathèse d'oléfines selon la revendication 1 ou 2, qui présente une structure chimique représentée par la formule (3) suivante

[Chem 5]

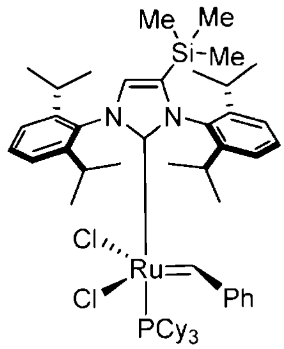

・・・(3)

dans la formule (3), Me représente un groupe méthyle, Ph représente un groupe phényle et $PCy_3$ représente la de tricyclohexylphosphine.

4. Utilisation du catalyseur complexe organométallique catalytique selon la revendication 1 pour des réactions de métathèse de fermeture de cycle de composés de dioléfines en chaîne.

5. Utilisation du complexe métallique organique catalytiques selon la revendication 1 pour des réactions de polymérisation par métathèse à ouverture de cycle de composés oléfiniques cycliques.

FIG. 1

$^1$H NMR spectrum (400 MHz, $C_6D_6$) of catalyst of Example 1

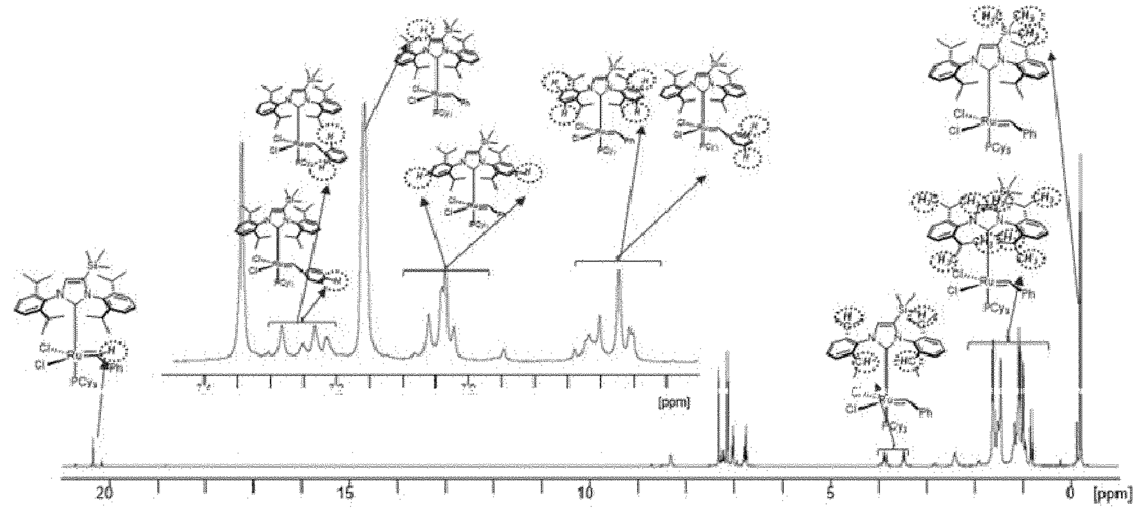


*The arrows in the drawing indicate that each peak in the $^1$H NMR spectrum belongs to which hydrogen among the plurality of hydrogens constituting the catalyst of Example 1.

FIG. 2

$^{29}Si$ NMR spectrum (119.2 MHz, $C_6D_6$) of catalyst of Example 1

Example 1
$^{TMS}$IPr-Ru complex catalyst

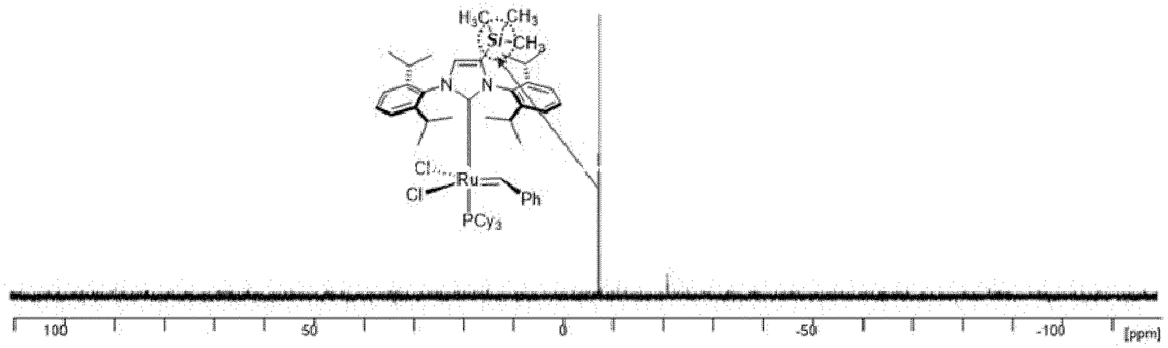

FIG. 3

$^{31}P$ NMR spectrum (162.1 MHz, $C_6D_6$) of catalyst of Example 1

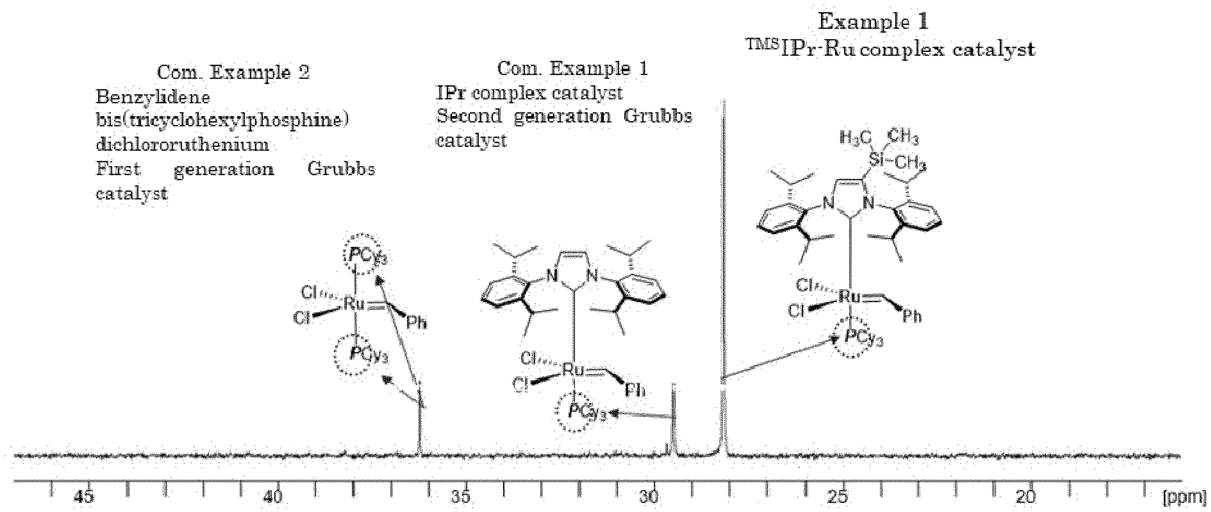

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP HEI11262667 A
- JP 2003500412 A
- JP 2002524250 A
- WO 2018105672 A

### Non-patent literature cited in the description


- **CESAR A. URBINA-BLANCO et al.** *J. Am. Chem. Soc.*, 2013, vol. 135, 7073-7079
- **P. SCHWAB et al.** *Angew. Chem., Int. Ed.*, 1995, vol. 34, 2039
- **JAFARPOUR, L. ; STEVENS, E. D. ; NOLAN, S. P.** *J. Organomet. Chem.*, 2000, vol. 606, 49-54
- **M. SCHOLL et al.** *Org. Lett.*, 1999, vol. 1, 953
- **T. DROGE ; F. GLORIUS**. *Angew. Chem. Int. Ed.*, 2010, vol. 49, 6940
- **WANG, Y et al.** *J. Am. Chem. Soc.*, 2010, vol. 132, 14370
- **TANG, P. ; WANG, W. ; RITTER, T.** *J. Am. Chem. Soc.*, 2011, vol. 133, 11482
- **POMPEO, M. ; FROESE, R. D. J. ; HADEI, N. ; ORGAN, M. G.** *Angew. Chem. Int. Ed.*, 2012, vol. 51, 11354
- **WANG, Y. ; XIE, YAMING. ; ABRAHAM, M. Y. ; WEI, P. ; SCHAEFER III, H. F. ; SCHLEYER, P. R. ; ROBINSON, G. H.** *J. Am. Chem. Soc.*, 2010, vol. 132, 14370